# EUROPÄISCHE PATENTANMELDUNG

(11) **EP 0 957 177 A2**
(43) Veröffentlichungstag der Anmeldung: **17.11.1999**
(21) Anmeldenummer: 99105604.5
(22) Anmeldetag: 19.03.1999
(51) Int. Cl.: C12Q 1/68

(54) **Verbessertes Verfahren zur Primer Extension Präamplifikations-PCR**

(30) Priorität: 26.03.1998 DE 19813317
(71) Anmelder: Roche Diagnostics GmbH, 68298 Mannheim (DE)
(72) Erfinder: Dietmaier, Wolfgang, Dr., 93049 Regensburg (DE); Rüschoff, Josef, Prof. Dr. med., 34119 Kassel (DE)

(57) **Zusammenfassung**

Die Erfindung betrifft einVerfahren zur Amplifikation von Nukleinsäurefragmenten aus einer Probe, das aus zwei oder drei thermozyklischen Amplifikationsreaktionen besteht, wobei während der ersten Amplifikationsreaktion vollständig randomisierte Primer verwendet werden und bei der zweiten Amplifikationsreaktion spezifische Primer verwendet werden, welches sich dadurch auszeichnet, daß zur Amplifikation der DNA eine Mischung aus mindestens zwei DNA Polymerasen eingesetzt wird, von denen mindestens eine Polymerase Proofreading Aktivität besitzt. Auf diese Weise kann DNA aus Einzelzellen oder Zellklonen mit geringer Zellzahl zur Mutationsanalyse verwendet werden.

## Beschreibung

Die Erfindung betrifft ein verbessertes Verfahren zur "Whole Genome Amplification" (WGA), welches dazu geeignet ist, eine DNA-Analyse ausgehend von einer oder nur wenigen Zellen durchführen zu können. Die Verbesserung der Methode wird im wesentlichen dadurch erreicht, daß zur Amplifikation der DNA eine Mischung aus zwei DNA-Polymerasen eingesetzt wird, von denen mindestens eine Polymerase 3'-5' Exonuklease-Aktivität besitzt.

WGA-Methoden sind von besonderer Bedeutung aufdem Gebiet der differentiellen Tumor-diagnostik. Zielsetzung einer differentiellen Tumordiagnostik auf molekularer Ebene ist die Analyse von Nukleinsäureproben aus Einzelzellen oder Zellpopulationen, die frei von nicht malignen Zellen sind (Emmert-Buck et al., 1996; Böhm und Wielang, 1997). Zur Gewinnung entsprechender Proben werden deshalb Zell-Sorting Methoden (Abeln et al., 1994, Barret et al., 1996) Mikrodissektionsmethoden (Shibata et al., 1992, Shibata et al., 1993, Emmert-Buck et al., 1994, Noguchi et al., 1994. Zhuang et al., 1995, Böhm et al., 1997) und die Methode der Lasermikrodissektion (Schütze und Clement-Sengewald, 1994) vermehrt eingesetzt.

Voraussetzung für die molekulare Analyse von Einzelzellen oder Populationen geringer Zellzahl sindjedoch besonders sensitive Methoden der Nukleinsäureamplifikation. Nach dem Stand der Technik sind dazu Methoden der "Whole Genome Amplification" (WGA) besonders geeignet. Dabei handelt es sich um Verfahren, die aus zwei aufeinanderfolgenden Amplifikationsreaktionen bestehen, wobei die erste Amplifikationsreaktion unter Verwendung von randomisierten Primern und die zweite Amplifikation unter Verwendung spezifischer Primer durchgeführt wird. Beispielsweise kann die WGA zur Diagnose von Erbkrankheiten bei der Präimplantationsdiagnostik von biopsierten Blastomerzellen (Kristianson et al., 1994, Snabes et al., 1994, van der Veyer et al., 1995) oder aber bei der Pränataldiagnostik von kernhaltigen Erythrozyten in maternalem Blut eingesetzt werden ( Sekizawa et al., 1996).

Nach dem Stand der Technik wird WGA bei Tumor-Biopsien in der Regel zur Analyse von Mikrosatelliten für den Nachweis von Mikrosatelliten-Instabilität oder Verlust an Heterozygotie in Tumorbiopsien eingesetzt. Die analysierte Probe muß dabei gemäß dem Stand der Technik jedoch soviele Zellen enthalten, daß eine mengenmäßig unverhältnismäßige Amplifikation einzelner Allele aufgrund zufälliger Präparationsartefakte ausgeschlossen ist (Zhang et al., 1992, Barret et al., 1995, Cheung und Nelson, 1996, Faulkner und Leigh, 1998). Beispielsweise wurden für eine Mikrosatellitenanalyse von FACS-sortierten aneuploiden Oesophagus-Tumorzellen in einem Ansatz etwa 1000 Zellen untersucht (Barret et al., 1995).

Im Gegensatz zur konventionellen in-situ Hybridisation (Van Ommen et al., 1995) oder zur konventionellen spezifischen PCR (Becker et al., 1996) ermöglichen Verfahren der WGA multiple Analysen der gleichen Probe. Dabei lassen sich zwei verschiedene Verfahren unterscheiden: Bei der "Degenerate Oligonukleotide Primer PCR" (DOP-PCR) werden Amplifikationsprimer mit definierten Sequenzen am 5' und am 3' Ende sowie mit einer randomisierten Hexamer-Region in der Mitte des Primers verwendet (Telenius et al., 1992). Ausgehend von wenig stringenten Bedingungen während der ersten 5 Thermozyklen werden die darauf folgenden 35 Thermozyklen unter stringenteren Bedingungen mit einer höheren Annealing-Temperatur durchgeführt, so daß während dieser Zyklen nur noch vollständig komplementäre Primer an die zu amplifizierende DNA binden können. Diese Verfahren werden beispielsweise als erster Schritt vor einer in-situ Hybridisation mit Flow-gesorteten Chromosomen (Blennow et al., 1992; Telenius et al., 1992; Kallionemie et al., 1994) oder auch zur Vergleichenden Genomischen Hybridisation CGH (Du-Manoir et al., 1993; Schlegel et al., 1995) angewandt.

Ein alternatives Prinzip der WGA ist die sogenannte "Primer Extension Preamplification" (PEP-PCR, Zhang et al., 1992). Im Gegensatz zur DOP-PCR werden komplett randomisierte 15 mer Amplifikationsprimer verwendet. Während 50 aufeinanderfolgenden Thermozyklen erfolgt zunächst eine Denaturierung bei 92°C und daran anschließend eine Hybridisierung unter wenig stringenten Temperaturbedingungen bei 37°C, die sukzessive mit einer Rate von etwa 0. 1°C/Sekunde auf 55° C erhöht wird. Bei dieser Temperatur erfolgt die Polymerase Extensionsreaktion für weitere 4 Minuten.

Sämtliche nach dem Stand der Technik bekannten Methoden (von Eggeling und Spielvogel, 1995) haben jedoch den Nachteil einer nicht ausreichenden Sensitivität, weil eine vergleichsweise große Zellzahl eingesetzt werden muß, um mit einem hohen Maß an Wahrscheinlichkeit ein Amplifikationsprodukt zu erhalten. Darüber hinaus wird die Sensitivität des Assays um so stärker reduziert, je länger das zu amplifizierende Fragment ist. Deshalb sind die nach dem Stand der Technik bekannten Methoden nur zur Amplifikation von vergleichsweise kleinen Fragmenten mit einer Länge von bis zu 580 Basenpaaren eingesetzt worden (Snabes et al., 1994).

Ein weiterer wesentlicher Nachteil der nach dem Stand der Technik bekannten PEP-PCR liegt insbesondere darin, daß aufgrund der inhärenten Fehlerrate der verwendeten Taq-Polymerase eine aussagekräftige DNA Mutationsanalyse bisher nicht zuverlässig durchgeführt werden konnte. Die Fehlerrate beruht darauf, daß die Verwendung von Taq-Polymerase während der Amplifikation zu AT/GC Transitionen im Amplifikationsprodukt führt (Keohvong and Thily, 1989). Darüber hinaus können bei Verwendung von Taq-Polymerase Deletionsmutationen entstehen, wenn die zu amplifizierende DNA zur Ausbildung von Sekundärstrukturen fähig ist (Carriello et al., 1991). Das Risiko des Erhalts von Amplifikationsartefakten ist bei der WGA jedoch besonders hoch, da in der Regel während der 2 bis 3 Amplifikationsreaktionen mehr als 80 Amplifikationszyklen durchgeführt werden.

Zur Vermeidung von Sequenzartefakten während der Nukleinsäureamplifikation war darüber hinaus nach dem Stand der Technik die Verwendung von DNA-Polymerasen mit 3'-5' Exonuklease-Aktivität bekannt (Flaman et al., 1994; Casas and Kirkpontrick, 1996). Der Einsatz von Polymerasen ohne 3'-5' Exonukleaseaktivität zur WGA führt jedoch zu einer weiteren Reduktion der Sensitivität des Verfahrens, da solche Polymerasen eine wesentlich geringere Prozessivität als beispielsweise Taq-DNA-Polymerase besitzen. Infolgedessen besitzen die unter Verwendung der randomisierten Primer während der Präamplifikation erzeugten Produkte keine hinreichende Länge, um als Matritze für die sich anschließende spezifische PCR-Reaktion dienen zu können, sofern das zu amplifizierende Fragment eine bestimmte Größe überschreitet.

Die zum Zeitpunkt der Erfindung zu lösende technische Aufgabe bestand demnach darin, ein Verfahren zu entwickeln, mit dessen Hilfe ausgehend von einer möglichst geringen Anzahl von Zellen spezifische Nukleinsäurefragmente in einer hohen Qualität, d.h. ohne Sequenzartekfakte amplifiziert und daran anschließend analysiert werden können. Die Qualität der Amplifikationsprodukte sollte zuverlässige Mutationsanalysen, Sequenzanalysen und eindeutig interpretierbare Mikrosatelliten Analysen ermöglichen. Diese Aufgabe wird gelöst durch ein verbessertes Verfahren zur Primer Extension preamplification (PEP-PCR, Zhang et al., 1992).

Gegenstand der Erfindung ist somit ein Verfahren zur Amplifikation von Nukleinsäurefragmenten aus einer Probe, das aus zwei oder drei thermozyklischen Amplifikationsreaktionen besteht, wobei während der ersten Amplifikationsreaktion vollständig randomisierte Primer verwendet werden und bei der zweiten Amplifikationsreaktion spezifische Primer verwendet werden, dadurch gekennzeichnet, daß zur Amplifikation der DNA eine Mischung aus mindestens zwei DNA-Polymerasen eingesetzt wird, von denen mindestens eine Polymerase 3'-5' Exonuklease-Aktivität besitzt. Diese Eigenschaft wird bei Polymerasen auch als Proofreading Aktivität bezeichnet (Flaman et al., 1994).

Eine Amplifikationsreaktion besteht aus ungefähr 20 bis 60 Thermozyklen, wobei während der ersten Amplifikationsreaktion bevorzugt mindestens 40 Thermozyklen, besonders bevorzugt aber mindestens 50 Thermozyklen durchgeführt werden. Für die zweite Amplifikationsreaktion werden bevorzugt mindestens 30 Thermozyklen, besonders bevorzugt aber mindestens 40 Thermozyklen durchgeführt.

Jeder Thermozyklus besteht aus einer Denaturierungsphase, einer Annealingphase sowie mindestens einer Elongationsphase. Vorzugsweise findet die Denaturierung zu Einzelsträngen bei Temperaturen zwischen 90°C und 96°C statt. Die Annealingphase zur Hybridisation der Primer an die Target-Nukleinsäure findet vorzugsweise bei Temperaturen zwischen 30°C und 50°C statt. Besonders bevorzugt findet die Annealingphase zwischen 35°C und 45°C statt. Während der ersten Amplifikationsreaktion findet die Annealingphase besonders bevorzugt bei ungefähr 37°C statt. Die Elongationsphase wird bei Temperaturen zwischen 50°C und 75°C durchgeführt. In einer bevorzugten Ausführungsform findet die Elongationsphase der ersten Amplifikationsreaktion bei Temperaturen zwischen 50°C und 60°C statt, wobei eine Temperatur von ungefähr 55°C als besonders bevorzugt wird.

Vorteilhaft für die Ausführung der beanspruchten Erfindung ist ein langsamer Übergang von der Annealingphase zur Elongationsphase, wobei dieser Übergang mit einer Geschwindigkeit von weniger als 0,5°C/Sekunde durchgeführt wird. Besonders vorteilhaft ist eine Durchführung der Temperaturtransition mit einer Geschwindigkeit von 0,1°C/Sekunde.

Für die Durchführung der erfindungsgemäßen Methode hat es sich auch als vorteilhaft erwiesen, wenn die Elongation während der ersten Amplifikationsreaktion in der Mehrzähl der Zyklen mit zwei oder mehr Elongationsschritten durchgeführt wird, wobei zunächst eine Elongation bei einer geringeren Temperatur und sodann die Fortsetzung der Elongation bei einer höheren Temperatur durchgeführt wird. Auf diese Weise werden während der ersten Amplifikationsreaktion Populationen von besonders langen Amplikons erzeugt. Vorzugsweise findet bei dieser Ausführungsform die erste Amplifikationsreaktion bei etwa 55°C und die zweite Amplifikationsreaktion bei ca 65°C bis 72°C statt, wobei sich eine Temperatur von ca. 68°C als optimal erwiesen hat.

Die in der ersten Amplifikationsreaktion verwendeten Primer sind vollständig randomisiert, d.h. es handelt sich um eine Population von einzelsträngigen Oligonukleotiden, bei denen jedes einzelne Nukleotid an jeder einzelnen Position aus einem der vier Nukleotidbausteine A. T, G oder C bestehen kann. Vorzugsweise besitzen diese Primer eine Länge von 10 - 20 Nukleotiden. Besonders bevorzugt sind Primer mit einer Länge von ca. 15 Nukleotiden. Die bei der zweiten Amplifikationsreaktion eingesetzten spezifischen Primer sind dadurch gekennzeichnet, daß sie eine Sequenz aufweisen, die über einen Bereich von mindestens 10 Nukleotiden identisch mit einer Sequenz der Target-Nukleinsäure bzw. deren komplimentärer Sequenz sind. Die bei einer eventuellen dritten Amplifikationsreaktion verwendeten spezifischen Primer zur Durchführung einer "nested PCR" werden nach den gleichen Kriterien ausgewählt wie die Primer der zweiten Amplifikationsreaktion, wobei die mit der Target-Nukleinsäure bzw. deren Komplement identischen Sequenzen der verwendeten Primer Bestandteil der in der zweiten Amplifikationsreaktion amplifizierten Sequenz sein müssen.

Die erfindungsgemäß eingesetzte Mischung aus DNA-Polymerasen enthält vorzugsweise eine thermostabile DNA-Polymerase ohne 3'-5' Exonukleaseaktivität wie beispielsweise Taq-DNA-Polymerase sowie eine weitere thermostabile DNA-Polymerase mit 3'- 5' Exonukleaseaktivität, wie beispielsweise die Pwo-DNA-Polymerase aus Pyrokokkus woesii (Boehringer Mannheim Katalog Nr. 1644947). Auch andere DNA-Polymerasen ohne 3'-5' Exonuktease-Aktivität können als Bestandteil der Polymerasenmischung eingesetzt werden.

In einer Ausführungsform des beanspruchten Verfahrens handelt es sich um ein Verfahren zur Amplifikation von DNA. Dabei hat es sich als vorteilhaft für die Sensitivität des Nachweises bestimmter Sequenzen erwiesen, zur Gewinnung der Proben-DNA die Zellyse des zu analysierenden Materials mit Hilfe eines enzymatischen Protease Verdaus durchzuführen. Beispielsweise kann dazu ProteinaseK verwendet werden.

In einer anderen Ausführungsform der erfindungsgemäßen Methode wird zunächst aus dem zu analysierenden Körpermaterial RNA isoliert, wobei von 1 Zelle, weniger als 10 Zellen oder weniger als 100 Zellen ausgegangen wird. Für die Gewinnung von RNA wird vorzugsweise eine chemische Lyse mit Guanidinum-Isothiocynat-haltigen Puffern eingesetzt. Mit Hilfe einer Reversen Transkriptase Reaktion wird anschließend eine entsprechende cDNA hergestellt, die anschließend als Ausgangsmaterial für die erfindungsgemäße Primer Extension Präamplification eingesetzt wird. Vozugsweise wird die cDNA durch reverse Transkription von Poly-A RNA erhalten.

Die beanspruchten Methoden sind zur Analyse von Proben aus Körpermaterial geeignet, welches aus einer oder nur wenigen Zellen besteht. Deshalb ist das beanspruchte Verfahren insbesondere auch zur Analyse von Nukleinsäuren aus Gewebsschnitten geeignet. Derartige Gewebeschnitte können gleichermaßen sowohl aus Gefriermaterial als auch durch Formalinfixiertes und in Paraffin gebettetes Gewebe erhalten werden. Besonders für diese Ausführungsformen ist ein entsprechender Protease-Verdau von Vorteil.

Der erfindungsgemäße Einsatz von Polymerasenmischungen zur Primer-Extension Präamplifikations-PCR führt zu einer überraschend hohen Sensitivität des Nachweises von DNA, die mit Hilfe der nach dem Stand der Technik bekannten Methoden nicht erreicht werden kann. Gegenstand der Erfindung ist demzufolge ein Verfahren zur Amplifikation von Nukleinsäurefragmenten, bestehend aus zwei oder drei thermozyklischen Amplifikationsreaktionen, wobei während der ersten Amplifikationsreaktion vollständig randomisierte Primer verwendet werden und bei der zweiten Amplifikationsreaktion spezifische Primer verwendet werden, wobei darüber hinaus die Probe eine Nukleinsäuremenge entsprechend einem Äquivalent von max. 100 Zellen enthält, welches dadurch gekennzeichnet ist, daß die Amplifikate mit einer Wahrscheinlichkeit von mehr als 90% gebildet werden. Insbesondere Gegenstand der Erfindung sind Verfahren, bei denen unter Einsatz eines Äquivalents von maximal 5 - 10 Zellen Amplifikate mit einer Wahrscheinlichkeit von mehr als 90% gebildet werden. In einer speziellen Ausführungsform werden bei Einsatz eines Zelläquivalents von einer Zelle Amplifikate mit einer Wahrscheinlichkeit von mehr als 50% gebildet.

Die erfindungsgemäße Methode ist zur Amplifikation von Nukleinsäurefragmenten mit einer Länge zwischen 100 und 1000 Basenpaaren geeignet. Besonders geeignet ist die Methode zur Amplifikation von Nukleinsäurefragmenten eine Länge von 150 und 550 Basenpaaren.

Zusammengefaßt ermöglicht die erfindungsgemäße Methode die Amplifikation spezifischer DNA-Fragmente aus Nukleinsäureproben, die aus nur einer bzw. wenigen Zellen gewonnen wurden, wobei gleichzeitig die Erzeugung von Amplifikationsartefakten ausgeschlossen ist oder zumindest minimiert wird. Gegenstand der Erfindung ist demzufolge ebenfalls die Verwendung einer erfindungsgemäß amplifizierten DNA zur Mutationsanalyse. Die Mutationsanalyse kann in einer besonderen Ausführungsform dadurch erfolgen, daß das erfindungsgemäß amplifizierte Nukleinsäurefragment mit Hilfe einer Sequenzierreaktion analysiert wird.

Gegenstand der beanspruchten Erfindung ist darüber hinaus die Verwendung der erfindungsgemäß amplifizierten DNA zur Analyse von Mikrosatelliten, insbesondere bei Analyse von Nukleinsäureproben, die aus Gerfrierschnitten oder Schnitten aus Formalin fixiertem und in Paraffin-eingebettetem Gewebe gewonnenen wurden. Vorzugsweise werden bei dieser Ausführungsform Zelläqivalente von 5-20 Zellen eingesetzt, da bei noch geringeren Zellmengen eine gleichmäßige Amplifikation von Allelen des gleichen Genlocus nicht mehr gewährleistet ist. Die erfindungsgemäße Analyse der Mikrosatelliten kann sowohl der Diagnose von Mikrosatelliten-Instabilität als auch zur Diagnose des Verlustes an Heterozygotie dienen (Boehm and Wieland, 1997).

Bei Einsatz eines Zelläquivalents von mindestens 10 Zellen ist darüber hinaus der Erhalt von Amplifikationsprodukten beider Allele mit einer Sicherheit von mehr als 90% gewährleistet.

### Kurzbeschreibung der Abbildungen

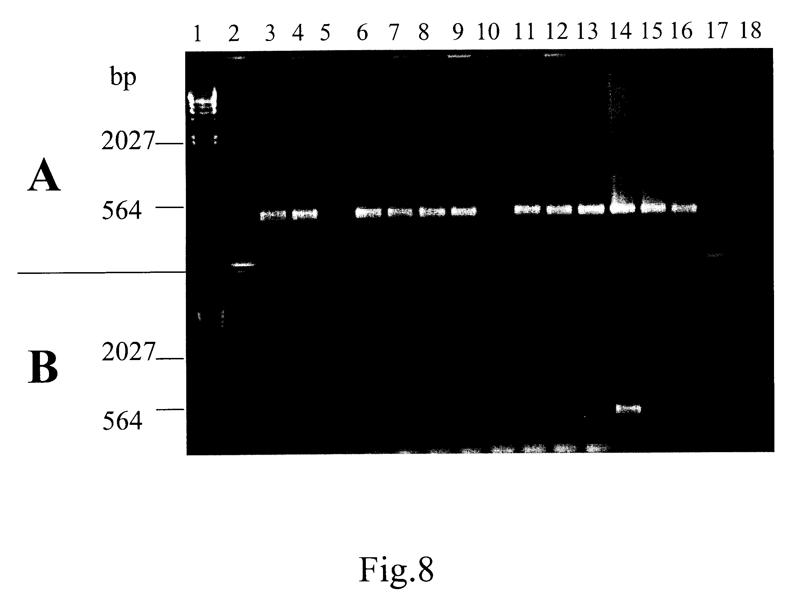

Abbildung 1: Amplifikation eines 536 bp Fragments des β-Globingens aus FACS-sortierten SW480 Zellen. Jeweils 10 Assays wurden mit 1, 5, 10 bzw. 100 Zellen durchgeführt.
Reihe A: Verwendung einer Expand-Polymerasenmischung nach Lyse mit ProteinaseK
Reihe B: Verwendung von Taq-Polymerase nach Lyse mit ProteinaseK
Reihe C: vorbekannte PEP-PCR nach alkalischer Lyse
Reihe D: DOP-PCR
Abbildung 2: Aus Gefrierschnitten einer Blasenkarzinombiopsie mikrodisseziertes Material in Aliquots von ca 50, 100, 200, 500 oder 1000 Zellen. Amplifikation eines 536 bp Fragments nach Lyse mit ProteinaseK (A) bzw. unter Verwendung von Taq-Polymerase nach alkalischer Lyse (B).
Abbildung 3: Mikrosatellitenanalyse des Locus D2S123 der Zelllinie SW480 mit jeweils 10 Proben, enthaltend 1, 5, 10 oder 100 Zellen.
Abbildung 4: Mikrosatellitenanalyse des Locus D2S123 aus mikrodisseziierten Gefrierschnitten einer Blasenkarzinombiopsie an Aliquots aus je ca 10, 25, 50 oder mehr als 1.000 Zellen.
Obere Gelreihe A: Erfindungsgemäße Präamplifikation, nach enzymatischer Lyse mit Expand-Polymerasenmischung.
Untere Gelreihe B: Präamplifikation mit Taq-Polymerase nach alkalischer Lyse
Abbildung 5: Mikrosatellitenanalyse des Locus D2S123 aus mikrodissoziierten Schnitten einer Colonkarzinombiopsie, die in Formalin fixiert und in Paraffin eingebettet wurden an Aliquots aus je ca 30, 50, 100 oder 250 Zellen.
Obere Gelreihe A: Erfindungsgemäße Präamplifikation, nach enzymatischer Lyse mit Expand-Polymerasenmischung.
Untere Geireihe B: Präamplifikation mit Taq-Polymerase nach alkalischer Lyse
Abbildung 6: Mutationsanalysen des P53-Gens anhand von erfindungsgemäß präamplifizierter DNA aus Mammakarzinombiopsien.
Abbildung 7: Nachweis von Ki-ras Mutationen an CK18-gefärbten, disseminierten Tumorzellen aus Knochenmark durch RFLP. DNA wurde jeweils nebeneinander unverdaut sowie mit MvaI verdaut aufgetragen.
   - Spuren 1,2,:: Immuncytochemisch CK18 positive Einzelzellen eines Pankreaskarzinompatienten
   - Spuren 4,5: : Immuncytochemisch CK18 positive Einzelzellen eines Colonkarzinompatienten
   - Spur 3,6,7:: Immuncytochemisch CK18 positive wenige Zellen eines Colonkarzinompatienten
   - Spuren 8-11:: jeweils etwa 100 ungefärbte hämapoetische Stammzellen als negative Kontrolle
Abbildung 8: Amplifikation eines 408 Bp Fragmentes des β2-Mikroglobulin-Gens unter Verwendung von aus Einzelzellen isolierter cDNA.
Oberes Gel: Spur 1: Größenmarker, HindIII verdaute Lambda-DNA; Spuren 2 bis 13: erfindungsgemäße Präamplifikations-PCR aus 12 verschiedenen Einzelzellen; Spur 14: positive Kontrolle mit erfindungsgemäß präamplifizierter cDNA aus 5.000 Zellen: Spur 15: positive Kontrolle mit 1 µg erfindungsgemäß präamplifzierter cDNA; Spur 16: positive Kontrolle mit 1 µg nicht präamplifizierter cDNA; Spur 17: negative Kontrolle ohne Reverse Transkriptase während der reversen Transkription; Spur 18: negative Kontrolle ohne Einsatz von cDNA während der Präamplifikation.
Unteres Gel: Spur1: Größenmarker, HindIII verdaute Lambda-DNA; Spuren 2 bis 13: 1/10 Aliquots von nicht präamplifizierter cDNA aus verschiedenen Einzelzellen; Spur 14: positive Kontrolle mit 1 µg nicht präamplifizierter cDNA.

### Beispiel 1:

### Sensitivität der erfindungsgemäßen Primer-Extension Präamplification

Zum Vergleich der erfindungsgemäßen Methode mit aus dem Stand der Technik bekannten Methoden wurden Zellen der Tumorzellinie SW480 (ATCC) mit Hilfe von Durchflußzytometrie in Äquivalente bestehend aus 1, 5, 10 oder 100 Zellen separiert. Zu diesem Zweck wurden die Zellen in PBS Puffer. enthaltend 0,1, µg/ml Fluoreseindiacetat resuspendiert und darin für 5 Minuten bei Raumtemperatur inkubiert. Dabei diffundiert das lipohile, nicht fluoreszierende Fluoreseindiacetat durch die Zellmembran und wird anschließend intrazellulär durch unspezifische Esterasen hydrolysiert,wodurch negativ geladenes, nicht mehr diffundierbares Fluorescein entsteht (Endl et al., 1996, Dive et al., 1990, Ross et al., 1989). Die Fluoreszenzmessungen wurden mit einem FACStarplus Zell-Sorter (Becton-Dickinson) durchgeführt. Dazu wurde die Fluoreszenz mit einem Wasser-gekühlten Argon-Ionen-Laser bei einer Wellenlänge von 488 nm angeregt. Die Emission wurde am FL1 Kanal unter Verwendung eines 525 nm Bandpassfilters mit einer Bandweite von 30 nm detektiert. Das Sorting wurde mithilfe von 20 Fluorescein-markierten Microbeads (Polysciences, Carboxy YG 4.5, Warrington) eingestellt. Die Ansätze wurden dreifach durchgeführt, wobei die Beads anschließend unter einem Fluoreszenzmikroskop ausgezählt wurden. Von einem korrekten Sorting wurde ausgegangen, sofern alle drei Sortiervorgänge zu einem Sortierergebnis von 20 Microbeads führten. Die Zellanalyse erfolgte mit einer Geschwindigkeit von etwa 200 Zellen pro Sekunde. Das Zell-Sorting erfolgte mit einer Tropfenschubgeschwindigkeit von 25 000 Hz direkt in Eppendorfgefäße,die bereits den verwendeten Lysispuffer enthielten. Dabei wurden jeweils 40 Aliquots von 1, 5, 10 oder 100 Zellen hergestellt.

Die Zelllyse erfolgte erfindungsgemäß in 10 µl High Fidelity Puffer (50 mM Tris-HCL, 22mM (NH₄)₂, SO₄ 2,5 mM MgCl₂, pH 8,9) zusätzlich enthaltend 4 mg/ ml ProteinaseK sowie 0,5 Vol% Tween 20 (Merck) während 12 Stunden bei 48°C, wobei im Anschluß daran eine Inaktivierung des Enzyms für 15 Minuten bei 94°C durchgeführt wurde. In Parallelansätzen erfolgte die Lyse gemäß dem Stand der Technik (Zhang et al. 1992) in 5 µl, 200 mM KOH, 50 mM Dithiothreitol für 10 Minuten bei 65°, wobei im Anschluß daran mit 5 µl 900 mM TrisHCl pH 8.3, 300 mM KCl neutralisiert wurde.

Anschließend wurde für jeweils 10 Proben eine erfindungsgemäße Präamplifkation unter Verwendung von vollständig randomisierten 15mer Primern (16 µM) und dNTP (100 µM) mit 5 Units einer Mischung aus Taq-Polymerase (Boehringer Mannheim) und Pwo-Polymerase (Boehringer Mannheim) im Verhältnis von 10:1 unter Standard-PCR-Pufferbedingungen (50 mM Tris-HCL, 22mM (NH₄)₂, SO₄, 2,5 mM Mgll₂, pH 8,9, zusätzlich enthaltend 0,05 mg/ml Gelatine) in einem Gesamtvolumen 60 µl mit folgenden 50 Thermozyklen durchgeführt:

| **Schritt** | **Temperatur** | **Zeit** |
|---|---|---|
| 1 | 92°C | 1′ 30˝ |
| 2 | 92°C | 40˝ |
| 3 | 37°C | 2′ |
| 4 | ramp: | 0,1°C/sec to 55°C |
| 5 | 55°C | 4′ |
| 6 | 68°C | 30˝ |
| 7 | gehe zu Schritt 2, 49 mal | |
| 8 | 8°C | 15′ |

Alternativ wurde die Präamplifikation mit folgenden 50 Thermozyklen durchgeführt:

| **Schritt** | **Temperatur** | **Zeit** |
|---|---|---|
| 1 | 94°C | 4′ |
| 2 | 94°C | 30˝ |
| 3 | 32°C | 1′ |
| 4 | ramp: | 0,1°C/sec to 55°C |
| 5 | 55°C | 45′′ |
| 6 | gehe zu Schritt 2, | |
| | 19 mal | |
| 7 | 94°C | 30′′ |
| 8 | 60° | 45′′ |
| 9 | ramp: | 1 °C/sec to 72°C |
| 10 | 72°C | 1′ |
| 11 | gehe zu Schritt 7 29 mal | |
| 12 | 72°C | 8min |
| 13 | 4°C | undefiniert |

Bei dieser Ausfürungsform wurde außerdem die Gelatine durch 5 Vol% DMSO ersetzt.

In Kontrollexperimenten wurden anstatt der erfindungsgemäß zu verwendenden Polymerasenmischung gemäß dem Stand der Technik 5 units Taq-Polymerase (Life Technologies) eingesetzt, wobei entweder durch alkalische Lyse oder aber durch enzymatische Lyse erhaltene Proben verwendet wurden. In einem weiteren Kontrollexperiment wurde nach enzymatischem ProteinaseK-Verdau Lyse eine DOP-PCR mithilfe des DOP-PCR Master Kits (Boehringer Mannheim) entsprechend dem Herstellerprotokoll durchgeführt.

In einer zweiten Amplifikationsrunde wurde ein Zehntel Aliquot (6 µl) nach Zugabe von je 0,5 µM Primern der Sequenz ID No. 1 und 2 ein 536 bp Fragment des β-Globin Gens in Gegenwart von 0,2 mM dNTP sowie 0,5 µl der jeweils gleichen Polymerase, die während der ersten Amplifikationsrunde verwendet wurde, in einem Gesamtvolumen von 20 µl unter PCR-Standard-Pufferbedingungen (High Fidelity 50 mM Tris-HCL pH 8,9, 22 mM (NH₄)₂ SO₄, 1.5 mM Mg Cl₂) und folgenden Thermozyklen spezifisch amplifiziert:

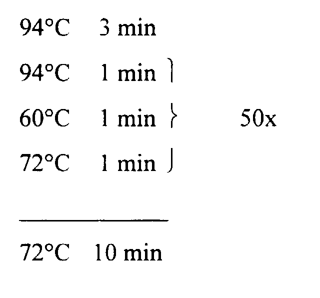

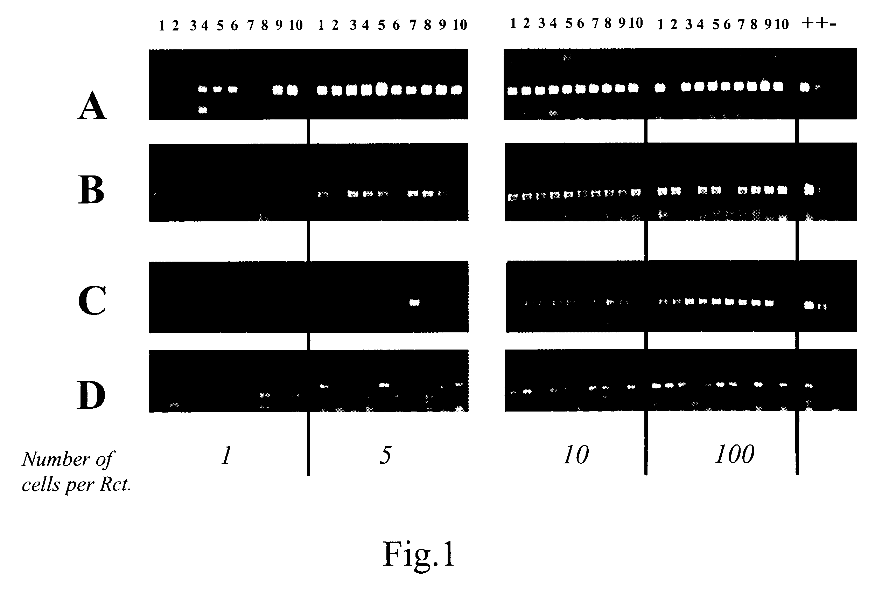

Die Amplifikationsprodukte wurden anschließend auf einem 2%igen Ethidiumbromid-gefärbten Agarosegel analysiert. Wie aus Abbildung 1 ersichtlich, ist die erfindungsgemäße Präamplifikation den Methoden der PEP-PCR bzw. der DOP-PCR bezüglich der Sensitivität bei der Amplifikation eines 536 Bp β-Globin-Fragments deutlich überlegen. In den Einzelzellassays wurde bei einer Präamplifikation unter Verwendung der erfindungsgemäßen Polymerasenmischung nach enzymatischer Lyse in mehr als 50% der untersuchten Zellen ein spezifisches Amplifikationsprodukt erhalten, wohingegen durch keines der anderen Verfahren ein Amplifikationsprodukt generiert werden konnte.

Bei den Assays mit 5 (oder mehr) Zellen führte die Verwendung der Polymerasenmischung nach enzymatischer Lyse bereits in allen getesten Proben zu einem Amplifikationsprodukt. Die Verwendung von Taq-Polymerase anstelle der Polymerasenmischung führte in 70% der in 5-Zell-Assays zu einem Amplifikationsprodukt. Im Gegensatz dazu wurden mithilfe der DOP-PCR oder der vorbekannten Präamplifikationsmethode. d.h. die Verwendung von Taq-Polymerase nach alkalischer Lyse, nur in der Minderzahl der getesteten Zellpopulationen Amplifikationsprodukte erhalten.

Daraus folgt, daß die erfindungsgemäße Verwendung einer Polymerasenmischung zur Präamplifikation bestehend aus Polymerase mit Proofreading-Aktivität (3'-5'-Exonuklease) und einer Polymerase ohne Proofreading-Aktivität, vorteilhaft bei der Analyse von kleinsten Zellpopulationen bis hin zu Einzelzellen ist, weil deren Verwendung mit einem hohen Maß an Wahrscheinlichkeit zu einem spezifischen Amplifikationsprodukt führt. Somit wird die Sensitivität derartiger Assays signifikant erhöht. Darüber hinaus zeigt das Experiment, daß für entsprechende Assays die Durchführung einer enzymatischen Zelllyse anstelle einer alkalischen Lyse von vorteilhafter Wirkung ist.

### Beispiel 2:

### Präamplifikation von aus Gefrierschnitten gewonnenem Zellmaterial

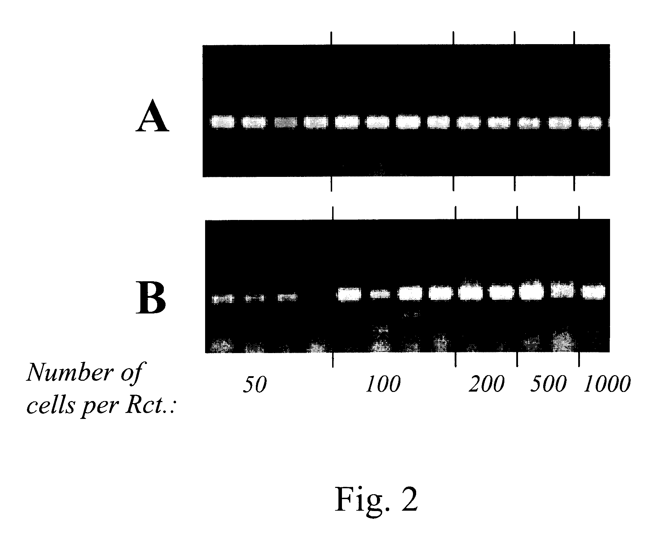

Aus einer Harnblasentumorbiopsie wurden 5µm dicke Gefrierschnitte hergestellt und mit Methylenblau angefärbt (Romeis. 1989). Daraus wurden Bereiche von ca 50, 100, 200, 500 oder 1000 Zellen mikrodisseziert. Die manuelle Mikrodissektion wurde unter Verwendung eines Mikromanipulators (Leitz; Serien Nr. 980629) und eines Invertmikroskops (Leitz, Labovert FS) bei 400-facher Vergrößerung. Anschließend erfolgte wie in Beispiel 1 beschrieben die Präamplifikation/Amplifikation eines 536 Bp β-Globin Fragments. Wie in Abbildung 2 dargestellt. erfolgt eine saturierte Amplifikation bei allen analysierten Aliquots, sofern die Präamplifikation erfindungsgemäß, d. h. unter Verwendung einer Expand-Polymerasenmischung nach Lyse mit ProteinaseK durchgeführt wird. Dagegen entstehen saturierte Amplifikationsprodukte bei der Verwendung von Taq-Polymerase nach alkalischer Lyse nur dann, wenn Aliquots von mindestens etwa 200 mikrodissoziierter Zellen eingesetzt werden.

### Beispiel 3:

### Sensitivität der Mikrosatellitenanalyse einer Tumorzellinie

Zellen der Zellinie SW480 (ATCC) wurden nach der in Beispiel 1 beschriebenen Methode in jeweils 10 Aliquots bestehend aus 1, 5, 10, bzw 100 Zellen separiert. Erfindungsgemäß erfolgte wie in Beispiel 1 beschrieben nach enzymatischer Lyse mit ProteinaseK eine Präamplifikation mit der Expand-Polymerase-Mischung.

Nach der Präamplifikation wurden 1/30 Aliquots für die Analyse des Mikrosatelliten-Locus D2S123 eingesetzt. Zur Amplifikation wurden 0,3 µM Primer der Seq. Id. No. 3 und 4 unter PCR-Standardbedingungen verwendet (Dietmaier et al., 1997). Die PCR-Amplifikationen wurden in einem Volumen von 20 µl wie folgt durchgeführt:
- Denaturierung: 94°C 1 Minute
- Annealing: 60°C 1 Minute
- Elongation: 72°C 1 Minute
Nach 50facher Wiederholung wurde der letzte Elongationsschritt bei 72°C für 8 Minuten durchgeführt.

3 µl des PCR-Produkts wurden mit 3 µl Probenpuffer (96% Formamid 1% Xylencyanol ff, 1% Brohmphenol Blau, 10 mMol EDTA pH 8.0) versetzt, für 4 Minuten bei 94°C denaturiert und auf einem 6,7%igen Polyacrylamid/50% Harnstoffgel für 1 Stunde bei 1.500 Volt und 50°C in einer Sequenziergelkammer (Biorad) gelelektrophoretisch aufgetrennt.

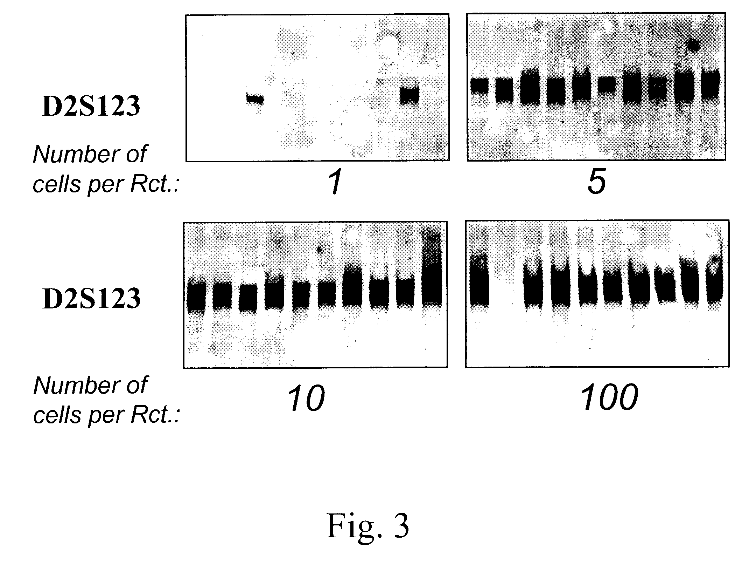

Wie aus Abbildung 3 ersichtlich, zeigten die Zellen der Mikrosatelliten-stabilen Zelllinie SW480 einen einheitlichen Alleltypus, wobei beide Allele bei einem Einsatz von mindestens 10 Zellen mit Sicherheit nachgewiesen werden konnten. In einem 5-Zell-Assay waren beide Allele noch mit etwa 80%iger Wahrscheinlichkeit detektierbar, im Einzelzell-Assay waren dagegen nur einzelne Allele mit einer geringen Erfolgsquote detektierbar.

### Beispiel 4:

### Mikrosatellitenanalyse an aus Gefrierschnitten gewonnenem Material

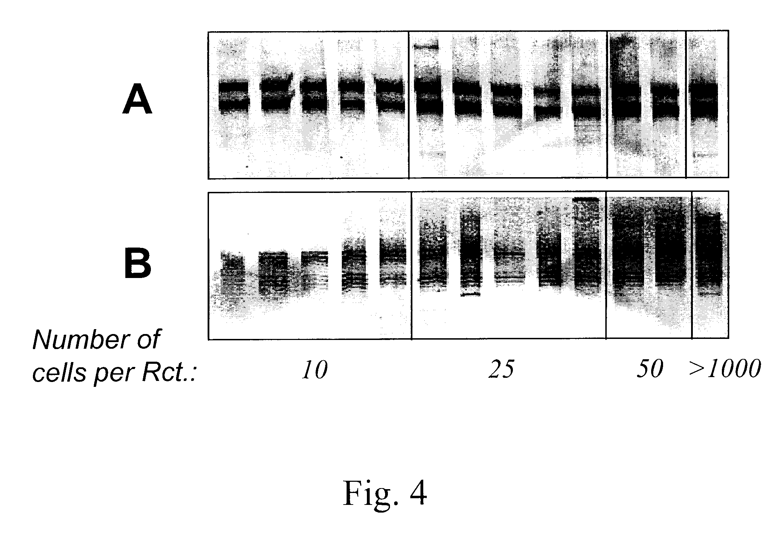

In einem anderem Experiment wurden eine Reihe von mikrodisseziierten Zellen einer Harnblasentumorbiopsie analog zu Beispiel 2 in Zelläquivalente zu jeweils ca. 10, 25, 50 und 1.000 Zellen separiert und entweder alkalisch oder aber mit ProteinaseK lysiert. Anschließend wurden die Lysate analog zu Beispiel 3 zur Mikrosatellitenanalyse des D2S123 Locus eingesetzt. Wie in Abbildung 4 dargestellt, reicht bei Durchführung einer erfindungsgemäßen Präamplifikations-PCR, d.h. bei enzymatischer Lyse mit ProteinaseK sowie Verwendung einer Enzymmischung aus Taq-Polymerase und Pwo-Polymerase ein Zelläqivalent von 10 Zellen bereits aus, um mit Sicherheit Allel-spezifische Amplifikationsprodukte zu erhalten. Beide Allele sind dabei gleichmäßig repräsentiert und erzeugen ein eindeutiges und auswertbares Bandenmuster. Dagegen führt die dem Stand der Technik gemäße Methode der alkalischen Lyse und anschließender Präamplifkation mithilfe von Taq-Polymerase bei der Analyse von etwa 10 Zelläquivalenten zu diffusen Bandenmustem, die nicht mehr eindeutig ausgewertet werden können.

### Beispiel 5:

### Mikrosatellitenanalyse von Material aus in Paraffin eingebettetem Gewebe

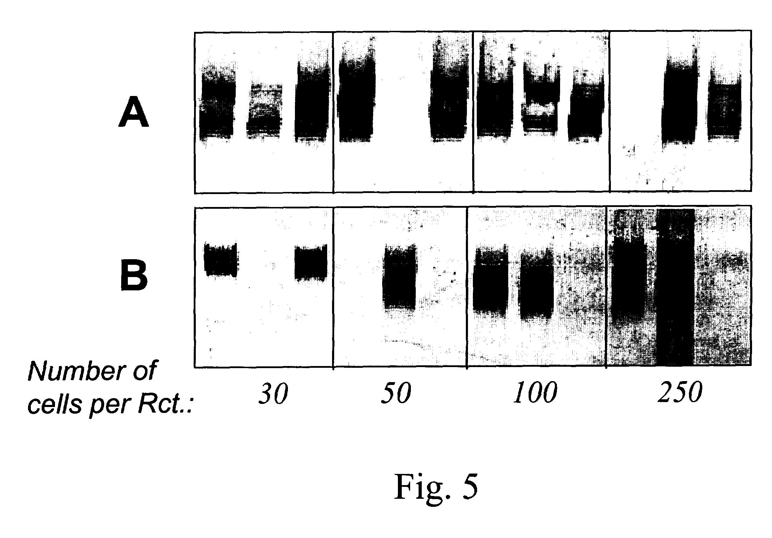

Colonkarzinomgewebe wurde mit Hematoxilin/Eosin angefärbt (Romers, 1989, S. 213), 14h in PBS-gepuffertem Formalin (Formalin: 3,7% Formaldehyd in PBS) fixiert, in Paraffin eingebettet und anschließend in 5 µm Schnitte zerlegt und auf Objekträger aufgetragen. Zur Entfärbung wurden die Objekträger für 2*15 min. in Xylen, 2*10 min. in 99,9% Ethanol, 2*10 min. in 96% Ethanol und 2*10 min. in 70% Ethanol gewaschen. Nach Mikrodissektion in etwa 30, 50, 100 und 250 Zellen wurde analog zu Beispiel 4 eine Mikrosatellitenanalyse durchgeführt. Wie in Abbildung 5 dargestellt, konnten bei Analyse eines Zelläqivalents von 30 Zellen durch eine erfindungsgemäße Präamplifikations-PCR, d.h. durch enzymatische Lyse mit ProteinaseK sowie Verwendung einer Enzymmischung aus Taq-Polymerase und Pwo-Polymerase, auswertbare Allel-spezifische Amplifikationsprodukte bei gleichmäßiger Repräsentation beider Allele erhalten werden. Dagegen führt die dem Stand der Technik gemäße Methode der alkalischen Lyse und anschließenden Präamplifikation mithilfe von Taq-Polymerase zu diffusen Bandenmustern, die häufig nur einzelne Allele repräsentrieren.

### Beispiel 6:

### P53-Mutationsanalyse

Um die Fehlerrate der Methode der verbesserten PEP-PCR mit anschließender Gen-spezifischer PCR-Amplifikation zu bestimmen, wurden genomische Regionen des P53 Gens (Exon7 bzw. Exon8) aus 8 verschiedenen Mammakarzinomen analysiert. Dazu wurden sogenannte "Touch Präparationen" (Kovach et al., 1991) hergestellt. Analog zu Beispiel 2 wurden Zellcluster von etwa 50-60 Zellen mikrodisseziert, die anschließend erfindungsgemäß enzymatisch lysiert und präamplifiziert wurden. Danach wurde eine P53-spezifische PCR durchgeführt. Als Kontrollreaktion wurde jeweils nicht präamplifizierte DNA unter den gleichen Bedingungen amplifiziert. Die spezifische Amplifikation erfolgte unter Standardbedingungen in einem Reaktionsvolumen von 50 µl (200nM dNTPs, 1,25 U Expand-Polymerase (Boehringer Mannheim), 1,5 mM (Exon7) bzw 2 mM (Exon8) MgCl₂ sowie jeweils 0,4 µM Amplifikationsprimern mit folgenden Thermozyklen:
- 1x: 94°C 2 min
- 35x: 94°C 1 min
50°C 2 min
72°C 3 min
- 1x: 72°C 10 min

Im Falle einer notwendigen nested PCR wurden 2 µl der ersten PCR-Reaktion eingesetzt. Folgende Primer wurden in verschiedenen Amplifikationsreaktionen eingesetzt:
- Seq. ID. No. 5: Exon7 first round up 5' AAAGGCCTCCCCTGCT 3'
- Seq. ID. No. 6: Exon7 first round down 5' GAGCAGTAAGGAGATT 3'
- Seq. ID. No. 7: Exon7 sec. round up 5' CTCCCCTGCTTGCCA 3'
- Seq. ID. No. 8: Exon7 sec. round down 5' GATGGGTAGTAGTATG 3'
- Seq. ID. No. 9: Exon8 first round up 5' GACAGGTAGACCTGAT 3'
- Seq. ID. No.10: Exon8 first round down 5' TCTGAGGCATAACTGC 3'
- Seq. ID No.11: Exon8 sec. round up 5' AGACCTGATTTCCTTAC 3'
- Seq. ID No.12: Exon8 sec. round down 5' TAACTGCACCCTTGGTC 3'

Die Amplifikationsprodukte wurden mit der Cycle-Sequencing Methode unter Verwendung des "PRISM ready dye terminator sequencing Kits" und AmpliTaqFS Polymerase (Applied Biosystems) sequenziert. Dazu wurden die amplifizierten DNA-Fragmente zunächst mit Polyethylenglykol präzipitiert. 50 - 150 ng des Präzipitats wurden mit je 3,2 pmol eines Sequenzier-Primers (Seq. ID. No. 5-12) und 8,0 µl Prä-mix, enthaltend Puffer, Farbstoff-markierte ddNTPs, dNTPs und Ampli-Taq-FS/Pyrophosphatase, versetzt. Nach einer Denaturierung bei 96° für 2 Min. wurden die Reaktionen über 25 Zyklen folgendermaßen inkubiert:
96°C/15 Sek..
50°C/15 Sek..
60°C/4 Min.

Anschließend wurden die Proben Ethanol-präzipitiert, getrocknet und in 2 µl Probenpuffer resuspendiert (5:1 deionisiertes Formamid, 0,05 Mol EDTA, PH8,), für 2 Minuten bei 92°C erhitzt und anschließend aufeinen Applied-Biosystem 373 Sequenzierapparat geladen.

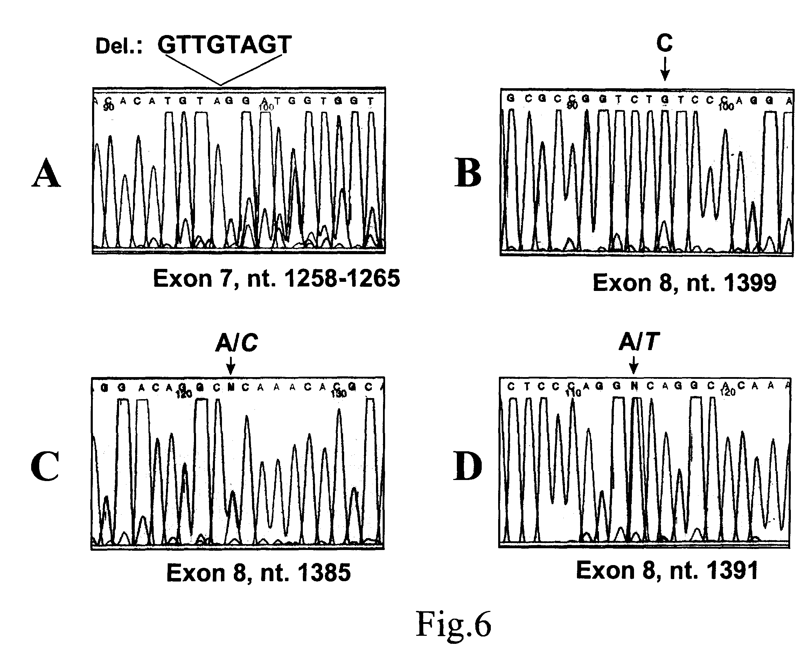

Ein Vergleich der Sequenzanalysen von erfindungsgemäß präamplifizierter DNA mit DNA des gleichen Patienten, welche direkt zur Sequenzierung eingesetzt wurde, ergab in keinem der untersuchten Patienten einen Unterschied. Die Position und die Art der identifizierten Mutationen sind in Abbildung 6 exemplarisch dargestellt. Abbildung 6 zeigt eine heterozygote 8-Basenpaardeletion in Exon7 (6a) eine hemozygote C/G-G/C-Transversion (6b) eine heterozygote A/T-/C/G-Transversion (6c) und eine heterozygote A/T-T/A-Transversion in Exon8 (6d) nach erfindungsgemäßer Primer-Extension Präamplifikation.Weitere Mutationen wurden nicht gefunden. Daraus folgt, daß eine erfindungsgemäße Präamplifikation mit einer Enzymischung aus Taq-Polymerase sowie einer Polymerase ohne Proofreadingaktivität nicht nur zu einer erhöhten Sensitivität von Amplifikationsreaktionen und Mikrosatellitenanalysen führt, sondern auch, daß die Amplifkationsprodukte hinsichtlich ihrer Sequenz keine Fehler aufweisen und deshalb zur Mutationsanalyse eingesetzt werden können.

### Beispiel 7:

### Analyse disseminierter Tumorzellen durch RFLP am Beispiel von Ki-ras

10 ml punktiertes Knochenmarksaspirat von Pankreastumorpatienten oder Kolonkarzinompatienten wurde über einen Ficoll-Dichtegradieten gereingt. Insgesamt 2x10⁶ Knochenmarkszellen wurden mit einem Anti-CK18-Antikörper (Klon CK2, Boehringer Mannheim) entsprechend dem Herstellerprotokoll immunhistochemisch angefärbt. CK18-positive Zellen wurden als Einzelzellen oder in Klustern mit sehr geringen Zellzahlen von den Zytospots mit einem Laser Mikrodissector (Schütze, 1994) mikrodissektiert. Dann wurde erfindungsgemäß analog zu Beispiel 1 enzymatisch mit ProteinaseK lysiert und eine Präamplifikation unter Verwendung einer Mischung aus Taq-Polymerase und Pwo-Polymerase (Mischungsverhältnis 10:1) durchgeführt.

Die Analyse des Restiktions-Fragment-Längenpolymorphismus erfolgte nach dem Protokoll von Trümper durch Anreicherungs-PCR (Trümper et al., 1994): dazu wurde ein 157 bp Fragment des Ki-ras Gens aus einem Zehntel des Ansatzes einer erfindungsgemäßen Präamplifikations PCR mit je 0,5 µM der Primer 5'BstNI (Seq. ID. No. 13) und 3'WT (Seq. ID. No. 14) in einem Volumen von 50 µl in 16 Thermozyklen bei einer Annealingtemperatur von 57°C mit 1U Expand HiFi Polymerase amplifiziert. Anschließend wurde jeweils ein 16 µl Aliquot in einem Volumen von 20 µl mit 20 U MvaI (BstNI Isoschizomer) verdaut. Jeweils 20 µl MvaI verdautes Fragment sowie 10 µl unverdautes Fragment wurden in einer zweiten PCR unter Verwendung von je 0,5µM 5'BstNI Primer sowie 3'BstNI Primer (Seq. ID. No. 15) in einem Gesamtvolumen von 50 µl während 35 Thermozyklen bei einer Annealingtemperatur von 60°C amplifiziert. Anschließend wurden 30 µl des Ansatzes mit MvaI verdaut und nach Zusatz von 7 µl nicht denaturierendem Probenpuffer gelelektrophoretisch auf einem nichtdenaturierenden 10%igen Polyacrylamidgel analysiert.

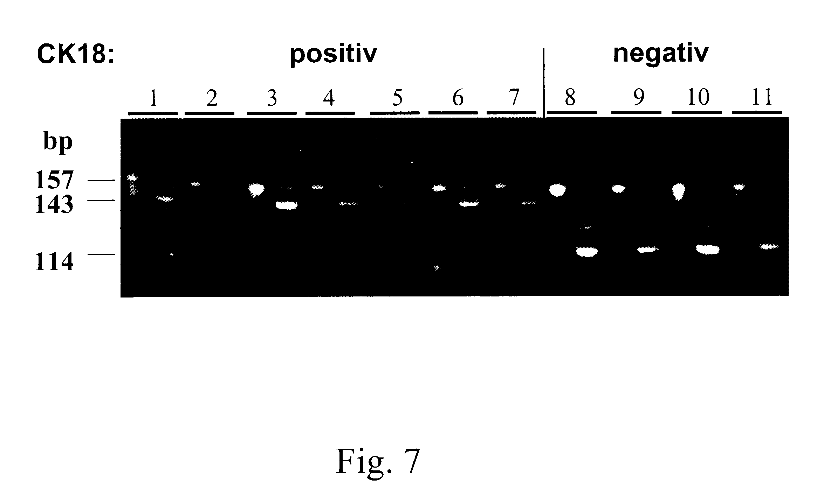

Wie in Abbildung 7 dargestellt, konnte DNA aus allen Proben erfolgreich amplifiziert werden. Die Existenz von DNA Fragmenten mit einer Größe von 143 bp nach MvaI Verdau weist die Mutation in sämtlichem untersuchtem Tumormaterial nach (Spuren 1-7). Sämtliche Kontrollreaktionen mit DNA aus nicht durch Anti-CK-18 anfärbbaren Zellen (Spuren 8-11) führen dagegen zu einem nicht mutierten 114 bp Produkt. Somit wurde erstmals eine Methode entwickelt, mit deren Hilfe mehrere Mutationen aus einer vereinzelten, disseminierten Tumorzelle mit einer hohen Nachweisequote zweifelsfrei diagnostiziert werden können.

### Beispiel 8:

### Präamplifikation von revers-transkribierter RNA

Kolorektalkarzinomzellen der Zellinie Lovo (ATCC) wurden mit Hilfe eines Fluoreszenzaktivierten Cell-Sorters vereinzelt. Aus den einzelnen Zellen wurde Poly (A) RNA mit Hilfe des Dynabeads mRNA Direct Kit (Dynal, Hamburg) isoliert. Der gesamte Ansatz wurde mit Hilfe von SuperScript RNaseH reverser Transkriptase (Life Technologies) unter Verwendung von Oligo(dT) Primern revers transkribiert (20 µl gemäß Hersteller). Die daraus resultierende cDNA (20 µl) wurde als Template für eine erfindungsgemäße Präamplifikation entsprechend dem unter Beispiel 1 beschriebenen Präamplifizierungs-PCR Protokoll eingesetzt. 1/30 der auf diese Weise präamplifizierten cDNA wurde anschließend als Template zur Amplifikation eines spezifischen 408 bp Fragmentes des β2-Microglobulingens eingesetzt. In einer entsprechenden Kontrollreaktion wurden jeweils 1/10 Aliquots der cDNA zur Amplifikation eingesetzt. Die PCR wurde dazu nach Standardbedingungen bei einer Annealingtemperatur von 60°C, 50 Zyklen sowie in Gegenwart von 0,2 mMol dNTP, 5 µMol PCR-Primern (Sequenz ID-Nr. 16 und 17) und 0.54 Units Expand Hifi-Polymerase in einem Volumen von 30 µl durchgeführt. Wie aus Abbildung 7 ersichtlich, konnte ein Amplifikationsprodukt aus präamplifizierter cDNA in 9 von 12 analysierten Einzelzellen erhalten werden, wohingegen die Verwendung von nicht erfindungsgemäß präamplifizierter DNA zu keinen nachweisbaren Amplifikationsprodukten führte.

### Referenzen:

Abeln ECA, Corver WE, Kuipers-Dijkshoorn NJ, Fleuren GJ and Cornelisse CJ (1994). Br J Cancer, 70, 255-262. Molecular genetic analysis of flow-sorted ovarian tumour cells: improved detection of loss of heterozygosity.
Barret MT, Galipeau PC, Sanchez CA, Emond MJ and Reid BJ. (1996). Oncogene, 12, 1873-1878. Determination of the frequency of loss of heterozygosity in esophageal adenocarcinoma by cell sorting, whole genome amplification and microsatellite polymorphisms.
Barret MT, Reid BJ and Geoffrey J. (1995). Nucleic Acids Research, 23, 3488-3492. Genotypic analysis of multiple loci in somatic cells by whole genome amplification.
Becker I, Becker K-F, Röhrl MH, Minkus G, Schütze K, and Höfler, H. (1996). Lab Invest. 75, 801-807. Single-cell mutation analysis of tumors from stained histologic slides.
Blennow E, Telenius H, Larsson C, de Vos D, Bajalica S, Ponder BA and Nordenskjold M (1992). Hum Genet, 90, 371-374. Complete characterization of a large marker chromosome by reverse and forward chromosome painting.
Böhm M, Wieland I, Schütze K and Rübben H. (1997). Am J Pathol, 15, 63-67. Microbeam MOMeNT. Non-contact laser microdissection of membrane-mounted native tissue.
Boehm M and Wieland I (1997). International J of Oncology, 10, 131-139. Analysis of tumor-specific alterations in native specimens by PCR: How to procure the tumour cells!
Casas E and Kirkpatrick BW. (1996). BioTechniques 20, 219-225. Evaluation of different amplification protocols for use in primer-extension preamplification.
Cheung V and Nelson SF (1996). Proc. Natl. Acad. Sci. USA, 93, 14676-14679. Whole genome amplification using a degenerate oligonucleotide primer allows hundreds of genotypes to be performed on less than one nanogram of genomic DNA.
Dietmaier W, Wallinger S, Bocker T, Kullmann F, Fishel, R, and Rüschoff J. (1997). Cancer Res., 57, 4749-4756. Diagnostic microsatellite instability: definition and correlation with mismatch repair protein expression.
Dive C, Watson JV and Workman P (1990). Cytometry 11, 244- 252. Multiparametric Analysis of cell membrane permeability by two colour flow cytometry with complementary fluorescent probes.
du-Manoir S, Speicher MR, Joos S, Schrock E. Popp S. Dohner H, Kovacs G, Robert-Nicoud M, Lichter P, and Cremer T. (1993). Hum-Genet. 1993, 90, 590-610. Detection of complete and partial chromosome gains and losses by comparative genomic in situ hybridization.
Emmert-Buck MR, Bonner RF, Smith PD, Chuaqui RF, Zhuang Z, Goldstein SR, Weiss RA, and Liotta LA (1996). Science 274, 998-1001. Laser capture microdissection.
Emmert-Buck-MR; Roth-MJ; Zhuang-Z; Campo-E; Rozhin-J; Sloane-BF; Liotta-LA; Stetler-Stevenson-WG (1994). Am J Pathol 145(6), 1285-1290. Increased gelatinase A (MMP-2) and cathepsin B activity in invasive tumor regions of human colon cancer samples.
Endl E, Steinbach P, Schärfe J, Fickweiler S, Wörle K. Hofstädter F (1996). Ultrasound. Med. Biol. 22, 515-525. Cell type specific response to shock waves of suspended or pelleted celts as analysed by flow cytometry or electrical cell volume determination.
Faulkner SW and Leigh A (1998). BioTechniques, 24, 47-50. Universal amplification of DNA from smal regions of paraffin-embedded formalin-fixed tissue.
Flaman JM, Freborg T, Moreau V, Charbonnier F, Martin C, Ishioka C, Friend SH and Iggo R (1994). Nucl Acids Res, 22, 3259-3260. A rapid PCR fidelity assay.
Kallioniemi A, Kallioniemi OP, Sudar D, Rutovitz D, Gray JW, Waldman F and Pinkel D (1992). Science, 258, 818-821. Comparative genomic hybridization for molecular cytogenetic analysis of solid tumors.
Keohvong P and Thily WG (1989). Proc. Natl. Acad. Sci. USA, 86, 9253-9257. Fidelity of DNA polymerases in DNA amplification.
Kovach JS, RM McGovern, Cassady JD, Swanson SK, Wold LE, Vogelstein B and SS Sommer (1991). Journal of the National Cancer Institute, 83, 1004-1009. Direct sequencing from touch preparations of human carcinomas: Analysis of p53 mutations in breast carcinomas.
Kristjansson K, Chong SS, Van den Veyer IB, Subramanian S, Snabes MC and Hughes MR. (1994). Nature Genetics, 6, 19-23. Preimplantation single cell analyses of dystrophin gene deletions using whole genome amplification.
Noguchi S; Motomura K; Inaji H; Imaoka S; Koyama H (1994). Cancer Res 54, 1849-1853. Clonal analysis of predominantly intraductal carcinoma and precancerous lesions of the breast by means of polymerase chain reaction.
Romeis B. 17. (1998). Mikroskopische Technik. 17.Aufl. Urban und Schwarzenberg, 1989. S.213-232.
Ross DD, Joneckis CC, Ordonez JV, Sisk AM. Wu RK. Hamburger AW and Nora RE (1989). Cancer Res 49, 3776-3782. Estimation of cell survival by flow cytometric quantification of fluorescein diacetate/ propidium iodide viable cell number.
Schütze K and Clement-Sengewald A (1994). Nature, 368, 667-669. Catch and move-cut or fuse.
Sekizawa A, Watanabe A, Kimura T, Saito H, Yanaihara T and Sato Takeshi. (1996). Obstetrics and Gynecology, 87 (4), 501-505. Prenatal diagnosis of the fetal RhD blood type using a single fetal nucleated erythrocyte from maternal blood.
Shibata D, Hawes D, Li ZH, Hernandez AM, Spruck CH and Nichols P. (1992). Am J Pathol. 141, 539-543. Specific genetic analysis of microscopic tissue after selective ultraviolet radiation fractionation and the polymerase chain reaction.
Shibata D. (1993). Am J Pathol, 143, 1523-1526. Selective ultraviolet radiation fractionation and polymerase chain reaction analysis of genetic alterations.
Snabes MC, Chong SS, Subramanian SB, Kristjansson K, DiSepio D and Hughes MR. (1994). Proc. Natl. Acad. Sci. USA, 91, 6181-6185.
Telenius H, Carter NP. Bebb CE. Nordenskjold M, Ponder BA and Tunnacliffe A (1992). Genomics. 1992, 13, 718-25. Degenerate oligonucleotide-primed PCR: general amplification of target DNA by a single degenerate primer.
Trümper LH, Bürger B, von Bonin F, Hintze A, von Blohn G. Pfreundschuh M and Daus H. (1994). Br. J. Cancer, 70, 278-284. Diagnosis of pancreatic adenocarcinoma by polymerase chain reaction from pancreatic secretions.
Van den Veyer IB. Chong SS, Cota JM, Bennett PR. Fisk NM, Handyside AH, Cartron J-P, Le Van Kim C, Colin Y, Snabes MC, Moise KJ, Hughes MR. (1995). Am J Obstet Gynecol, 172, 533-540. Single-cell analysis of the RhD blood type for use in preimplantation diagnosis in the prevention of sever hemolytic disease of the newborn.
Van Ommen GJ, Breuning MH and Raap-AK (1995). Curr Opin Genet Dev, 5(3): 304-308. FISH in genome research and molecular diagnostics.
Von Eggeling F and Spielvogel H (1995). Cell and Mol Biology, 41(5), 653-670. Applications of random PCR.
Zhang L, Cui X, Schmitt K, Hubert R, Navidi W, and Arnheim N (1992). Proc. Natl. Acad. Sci. USA, 89, 5847-5851. Whole genome amplification from a single cell: Implications for genetic analysis.
Zhuang Z, Bertheau P, Emmert-Buck MR, Liotta LA, Gnarra J, Linehan WM, Lubensky IA. (1995). Am J Pathol, 146, 620-625. A microdissection technique for archival DNA analysis of specific cell populations in lesions <1mm in size.

## Patentansprüche

1. Verfahren zur Amplifikation von Nukleinsäurefragmenten aus einer Probe, das aus zwei oder drei thermozyklischen Amplifikationsreaktionen besteht, wobei während der ersten Amplifikationsreaktion vollständig randomisierte Primer verwendet werden und bei der zweiten Amplifikationsreaktion spezifische Primer verwendet werden, dadurch gekennzeichnet,
daß zur Amplifikation der DNA eine Mischung aus mindestens zwei DNA Polymerasen eingesetzt wird, von denen mindestens eine Polymerase 3'-5' Exonuklease-Aktivität besitzt.

2. Verfahren nach Anspruch 1, dadurch gekennzeichnet,
daß während der ersten thermozyklischen Amplifikationsreaktion in der Mehrzahl der Zyklen zwei oder mehrere Elongationsschritte bei verschiedenen Temperaturen durchgeführt werden.

3. Verfahren nach Anspruch 1-2, dadurch gekennzeichnet,
daß die Probe durch eine Zell-Lyse mit Hilfe eines enzymatischen Protease-Verdaus gewonnen wird.

4. Verfahren nach Anspruch 1-2, dadurch gekennzeichnet,
daß die Probe aus einem Pool von cDNAs besteht.

5. Verfahren nach Anspruch 1-4, dadurch gekennzeichnet, daß
die eingesetzte Nukleinsäure aus Gewebeschnitten gewonnen wird.

6. Verwendung von DNA, die nach Anspruch 1-5 amplifiziert wurde, zur Mutationsanalyse.

7. Verwendung von DNA, die nach Anspruch 1-5 amplifiziert wurde, als Matritze für Sequenzierreaktionen.

8. Verwendung von DNA, die nach Anspruch 1-5 amplifiziert wurde, zur Analye von Mikrosatelliten.

9. Verfahren zur Amplifikation von Nukleinsäurefragmenten aus einer Probe, das aus zwei oder drei thermozyklischen Amplifikationsreaktionen besteht, wobei während der ersten Amplifikationsreaktion vollständig randomisierte Primer verwendet werden und bei der zweiten Amplifikationsreaktion spezifische Primer verwendet werden, wobei die Probe eine Nukleinsäuremenge entsprechend einem Äquivalent von maximal 100 Zellen enthält, dadurch gekennzeichnet, daß die Amplifikate mit einer Wahrscheinlichkeit von mehr als 90 % gebildet werden.

10. Verfahren nach Anspruch 9, wobei als Probe eine Nukleinsäuremenge entsprechend einem Äquivalent von maximal 10 Zellen eingesetzt wird, dadurch gekennzeichnet, daß die Amplifikate mit einer Wahrscheinlichkeit von mehr als 90 % gebildet werden.

11. Verfahren nach Anspruch 9, wobei als Probe eine Nukleinsäuremenge entsprechend einem Äquivalent von maximal 5 Zellen eingesetzt wird, dadurch gekennzeichnet, daß die Amplifikate mit einer Wahrscheinlichkeit von mehr als 90 % gebildet werden.

12. Verfahren nach Anspruch 9, wobei als Probe eine Nukleinsäuremenge entsprechend einem Äquivalent von einer Zelle eingesetzt wird, dadurch gekennzeichnet, daß die Amplifikate mit einer Wahrscheinlichkeit von mehr als 50 % gebildet werden.

13. Verfahren zur Analyse von Mikrosatelliten, welches aus zwei oder drei thermozyklischen Amplifikationsreaktionen besteht, wobei während der ersten Amplifikationsreaktion vollständig randomisierte Primer verwendet werden und bei der zweiten Amplifikationsreaktion spezifische Primer verwendet werden, wobei die Probe eine Nukleinsäuremenge entsprechend einem Äquivalent von maximal 100 Zellen enthält, dadurch gekennzeichnet, daß die Amplifikate beider Allele mit einer Wahrscheinlichkeit von mehr als 90 % gebildet werden.

14. Verfahren nach Anspruch 13, wobei als Probe eine Nukleinsäuremenge entsprechend einem Äquivalent von maximal 10 Zellen eingesetzt wird. dadurch gekennzeichnet, daß die Amplifikate mit einer Wahrscheinlichkeit von mehr als 90 % gebildet werden.

15. Verfahren nach Anspruch 13, wobei als Probe eine Nukleinsäuremenge entsprechend einem Äquivalent von maximal 5 Zellen eingesetzt wird, dadurch gekennzeichnet, daß die Amplifikate beider Allele mit einer Wahrscheinlichkeit von mehr als 50 % gebildet werden.


SEQUENZPROTOKOLL

(1) ALLGEMEINE ANGABEN:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 68305
(G) TELEFON: 06217591456
(H) TELEFAX: 06217594457

(ii) BEZEICHNUNG DER ERFINDUNG: Verbessertes Verfahren zur Primer
Extension Präamplifikations-PCR

(iii) ANZAHL DER SEQUENZEN: 17

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)


(2) ANGABEN ZU SEQ ID NO: 1:

(i) SEQUENZKENNZEICHEN:
(A) LÄNGE: 22 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 1:

GGTTGGCCAA TCTACTCCCA GG
22

(2) ANGABEN ZU SEQ ID NO: 2:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 2:

GCTCACTCAG TGTGGCAAAG
20

(2) ANGABEN ZU SEQ ID NO: 3:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 3:

AAACAGGATG CCTGCCTTTA
20

(2) ANGABEN ZU SEQ ID NO: 4:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 4:

GGACTTTCCA CCTATGGGAC
20

(2) ANGABEN ZU SEQ ID NO: 5:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 5:

AAAGGCCTCC CCTGCT
16

(2) ANGABEN ZU SEQ ID NO: 6:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 6:

GAGCAGTAAG GAGATT
16

(2) ANGABEN ZU SEQ ID NO: 7:

(i) SEQUENZKENNZEICHEN:
(A) LÄNGE: 15 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 7:

CTCCCCTGCT TGCCA
15

(2) ANGABEN ZU SEQ ID NO: 8:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 8:

GATGGGTAGT AGTATG
16

(2) ANGABEN ZU SEQ ID NO: 9:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 9:

GACAGGTAGA CCTGAT
16

(2) ANGABEN ZU SEQ ID NO: 10:

(i) SEQUENZKENNZEICHEN:
(A) LÄNGE: 16 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 10:

TCTGAGGCAT AACTGC
16

(2) ANGABEN ZU SEQ ID NO: 11:

(i) SEQUENZKENNZEICHEN:
(A) LÄNGE: 17 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 11:

AGACCTGATT TCCTTAC
17

(2) ANGABEN ZU SEQ ID NO: 12:

(i) SEQUENZKENNZEICHEN:
(A) LÄNGE: 17 Basenpaare
(B) ART: Nucleotid
(C) STRANGFORM: Einzelstrang
(D) TOPOLOGIE: linear

(ii) ART DES MOLEKÜšLS: Genom-DNA

(xi) SEQUENZBESCHREIBUNG: SEQ ID NO: 12:

TAACTGCACC CTTGGTC
17

(2) ANGABEN ZU SEQ ID NO: 13:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 13:

ACTGAATATA AACTTGTGGT AGTTGGACCT
30

(2) ANGABEN ZU SEQ ID NO: 14:

(i) SEQUENZKENNZEICHEN:

(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 14:

TCAAAGAATG GTCCTGCACC
20

(2) ANGABEN ZU SEQ ID NO: 15:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 15:

TCAAAGAATG GTCCTGGACC
20

(2) ANGABEN ZU SEQ ID NO: 16:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 16:

GGCATTCCTG AAGCTGACAG C
21

(2) ANGABEN ZU SEQ ID NO: 17:

(i) SEQUENZKENNZEICHEN:
(A) LÄNGE: 23 Basenpaare
(B) ART: Nucleotid
(C) STRANGFORM: Einzelstrang
(D) TOPOLOGIE: linear

(ii) ART DES MOLEKÜLS: Genom-DNA

(xi) SEQUENZBESCHREIBUNG: SEQ ID NO: 17:

CTCCATGATG CTGCTTACAT GTC
23